# DEMANDE DE BREVET EUROPEEN

(11) **EP 3 141 228 A1**
(43) Date de publication de la demande: **15.03.2017**
(21) Numéro de dépôt: 16001753.9
(22) Date de dépôt: 08.08.2016
(51) Int. Cl.: A61F 9/02, G02C 5/00

(54) **MASQUE DE SPORT**

(30) Priorité: 10.08.2015 FR 1501702
(71) Demandeur: Salomon S.A.S., 74370 Metz-Tessy (FR)
(72) Inventeur: Donnadieu, Thierry, 74330 Sillingy (FR); Girault, Eric, 74000 Annecy (FR)

(57) **Abrégé**

Masque de protection oculaire (1) pour la pratique de sports en plein air comprenant :
- un écran déformable (3),
- un châssis rigide (2) comprenant au moins un logement d'accroche (27a, 27b) destiné à coopérer avec l'écran afin de maintenir solidaire l'écran avec le châssis, selon au moins une direction d'assemblage (X),
caractérisé en ce que
le logement d'accroche est apte à coopérer avec l'écran uniquement lorsque l'écran est déformé,
et en ce que
le masque comprend au moins un moyen de maintien (4, 5) permettant de maintenir la déformation de l'écran lorsque celui-ci est assemblé sur le châssis.

## Description

L'invention concerne les masques de protection oculaire, en particulier les masques de protection pour la pratique de sports en plein air, tels que le ski ou surf alpin, le vélo tout terrain ou le motocross, à l'exclusion des sports nautiques, en particulier les lunettes de natation, du fait que l'usage est complètement différent.

Des masques de protection sont habituellement utilisés durant la pratique de certains sports de plein air pour protéger les yeux de l'utilisateur du vent, de l'eau, de la boue ou du rayonnement solaire.

Un tel masque comprend généralement un châssis supportant un écran de protection transparent et une sangle fixée de part et d'autre du châssis. La sangle permet de maintenir le masque sur le visage en entourant partiellement le crâne de l'utilisateur. Des éléments de confort sont habituellement ajoutés au châssis pour améliorer le confort du port du masque. Ces éléments de confort sont placés à l'interface entre le châssis et le visage. Ils forment souvent une bande de mousse entourant les yeux en prenant appui sur une partie du front, les tempes, les pommettes et une partie du nez de l'utilisateur.

L'utilisateur peut souhaiter remplacer l'écran pour diverses raisons. Par exemple, il peut vouloir changer l'indice de filtration des rayonnements solaires, en fonction des conditions météorologiques. Il peut aussi remplacer un écran endommagé. Ce peut être aussi pour des raisons esthétiques, l'utilisateur voulant un écran correspondant aux dernières tendances de la mode.

Pour répondre à ce besoin, il existe de nombreuses solutions connues assurant cette interchangeabilité. Beaucoup de ces solutions sont complexes, nécessitent une multitude de pièces et ne sont pas toujours ergonomiques.

Une construction basique consiste à utiliser un châssis souple, généralement en polyuréthanne, incorporant une rainure sur tout le pourtour interne d'une ouverture délimitant le champ visuel. L'écran est alors inséré dans la rainure par déformation du châssis. L'écran assemblé n'est pas maintenu déformé sur le châssis. La mise en place de l'écran n'est pas facile.

Une deuxième construction consiste à utiliser un écran muni de clips venant coopérer avec un châssis. Lorsque l'écran est assemblé sur le châssis, les clips reprennent leurs positions initiales. L'écran n'est alors plus sollicité en déformation. Pour retirer l'écran, il faut alors exercer un effort directement sur l'écran ou sur le clip, ou encore déformer l'armature. Ce n'est pas une opération très pratique et facile. De plus, le dimensionnement du clip est généralement petit ce qui ne facilite pas la manipulation. Une telle construction est illustrée dans le document US 2009/0222979.

Un autre mode de réalisation consiste à mettre en place l'écran dans un logement ménagé sur un châssis, puis à actionner un verrou assurant le maintien de l'écran. Ce type de masque est décrit dans les documents US2004/025232, US 2014/0033408 ou US2009/038059.

Une autre variante consiste à fixer l'écran grâce à des aimants. Cette solution est illustrée dans le document US2013/0185849.

Dans toutes les solutions proposées dans l'état de la technique, le masque n'est pas conçu pour maintenir l'écran déformé sur le châssis lorsque le masque est assemblé.

Le but de l'invention est de proposer une solution alternative pour fixer un écran de manière amovible sur un châssis d'un masque.

Un autre but est de proposer un masque dont la mise en place et le retrait de l'écran sont simples et ergonomiques.

Un autre but est de proposer un masque constitué d'un nombre réduit de pièces.

L'invention propose un masque de protection oculaire pour la pratique de sport en plein air comprenant :
- un écran déformable,
- un châssis rigide comprenant au moins un logement d'accroche destiné à coopérer avec l'écran afin de maintenir solidaire l'écran avec le châssis, selon au moins une direction d'assemblage.

Le masque est caractérisé par le fait que le logement d'accroche est apte à coopérer avec l'écran uniquement lorsque l'écran est déformé et que le masque comprend au moins un moyen de maintien permettant de maintenir la déformation de l'écran lorsque celui-ci est assemblé sur le châssis.

Grâce à cette construction, le logement d'accroche n'interfère pas avec l'écran lorsque celui-ci n'est pas déformé. La mise en place de l'écran est donc facilitée. La conception du masque peut être également simplifiée du fait que le logement d'accroche peut être simple et basique. Le logement d'accroche peut être directement intégré sur le châssis ce qui réduit le nombre de pièces constitutives.

Selon des aspects avantageux mais non obligatoires de l'invention, un tel masque peut incorporer une ou plusieurs des caractéristiques suivantes, prises dans toute combinaison techniquement admissible :
- le châssis est constitué d'un matériau présentant un module d'élasticité supérieur à 1 000 MPa,
- le châssis est constitué d'un matériau parmi le PolyAmide (PA), le PolyCabonate (PC), l'Acrylonitrile Butadiène Styrène (ABS),
- le châssis comprend une surface d'appui contre lequel vient s'appuyer l'écran et en ce que le logement d'accroche est délimité par une partie de la surface d'appui et une paroi, disposée en vis-à-vis de la partie de la surface d'appui, à une distance,
- la surface d'appui présente un rayon de courbure supérieur au rayon de courbure de l'écran,
- le logement d'accroche comprend un aménagement destiné à coopérer avec une forme de l'écran de sorte à maintenir solidaire l'écran avec le châssis selon une direction différente de la direction d'assemblage,
- le au moins un moyen de maintien comprend un clip,
- le au moins un moyen de maintien est disposé au centre d'un montant supérieur du châssis,
- le masque comprend un deuxième moyen de maintien disposé au centre d'un montant inférieur du châssis,
- le masque comprend deux logements d'accroches disposés respectivement sur un montant latéral du châssis,
- l'écran et le châssis sont dimensionnés de sorte que chaque bord latéral de l'écran se déforme transversalement, entre une position libre de l'écran et une position de l'écran assemblé sur le châssis, d'une distance supérieure à deux millimètres,
- la largeur de l'écran non déformé, correspondant à la distance transversale séparant les deux bords latéraux, est toujours inférieure à la distance transversale séparant les parois externes délimitant respectivement les logements d'accroche,
- l'écran couvre donc les deux yeux de l'utilisateur lorsqu'il est assemblé sur le châssis,
- l'écran comprend des inserts s'étendant depuis la face arrière de l'écran, chaque insert étant destiné à coopérer avec le logement d'accroche correspondant, lorsque l'écran est déformé, de sorte à maintenir solidaire l'écran avec le châssis, selon au moins une direction d'assemblage,
- la face arrière de l'écran comprend une zone périphérique destinée à venir en contact avec une surface d'appui du châssis, la zone périphérique étant prévue pour épouser sensiblement la forme de la surface d'appui lorsque l'écran est déformé.

D'autres caractéristiques et avantages de l'invention seront mieux compris à l'aide de la description qui va suivre, en regard des dessins annexés illustrant, selon des formes de réalisation non limitatives, comment l'invention peut être réalisée, et dans lequel :

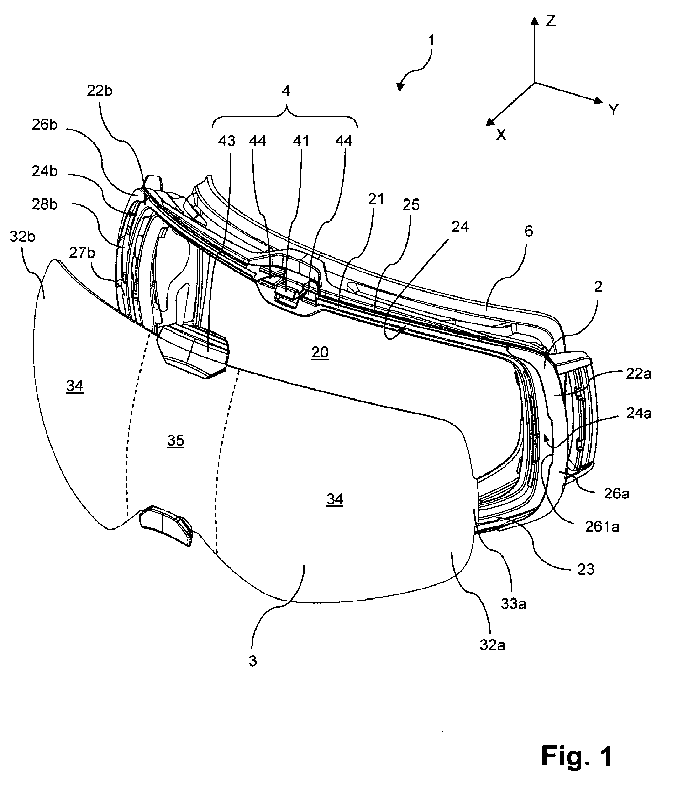

- la figure 1 est une vue éclatée en perspective avant du masque selon l'invention ;

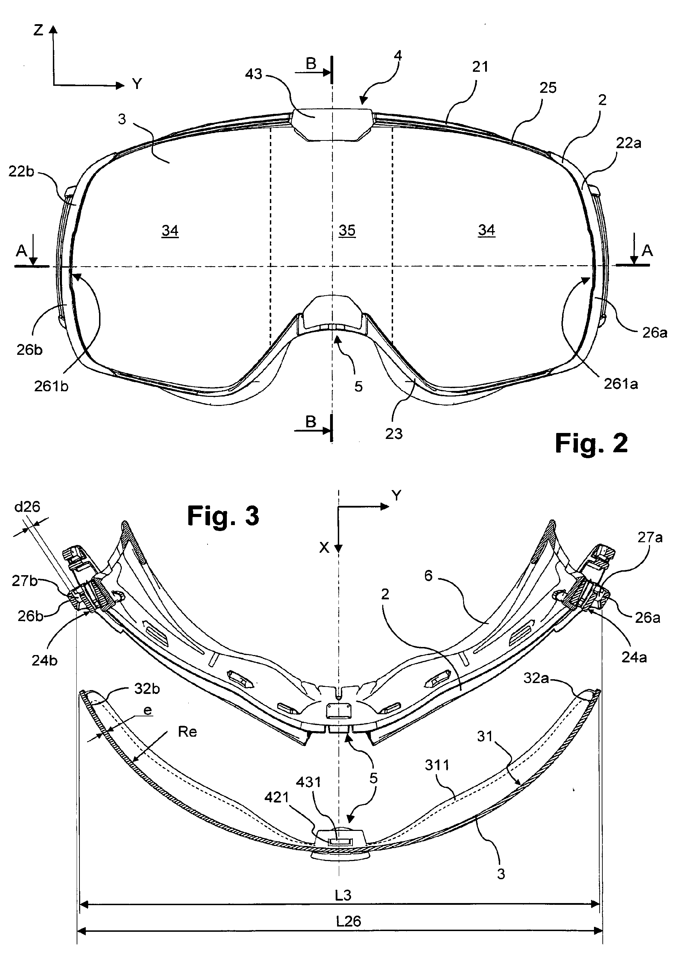

- la figure 2 est une vue de face du masque assemblé ;
- les figures 3, 4 et 5 sont des vues en coupe selon A-A de la figure 2 illustrant les différentes étapes d'assemblage de l'écran sur le châssis ;

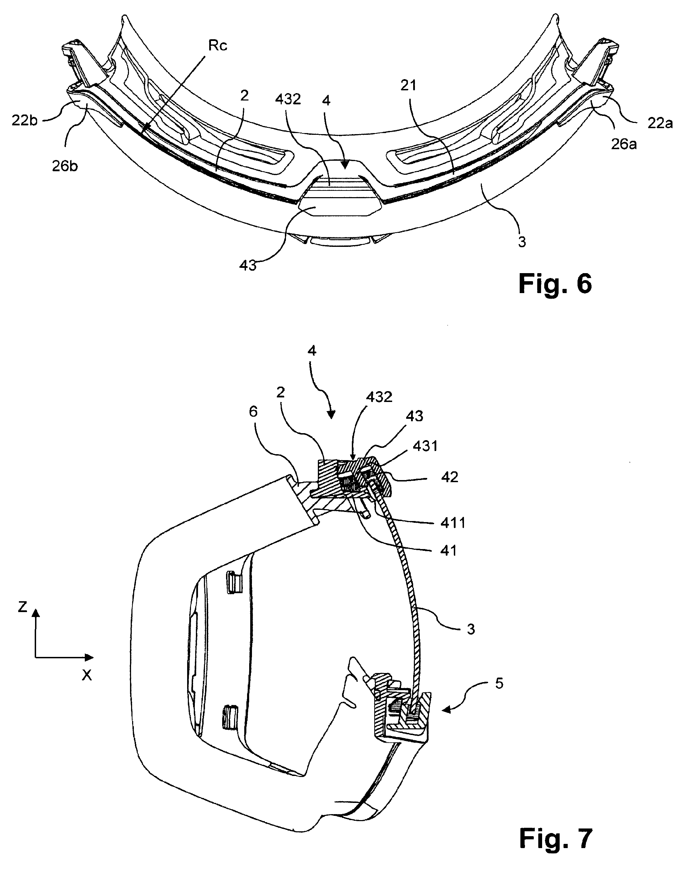

- la figure 6 est une vue de dessus du masque assemblé ;
- la figure 7 est une vue en coupe selon B-B de la figure 2 ;

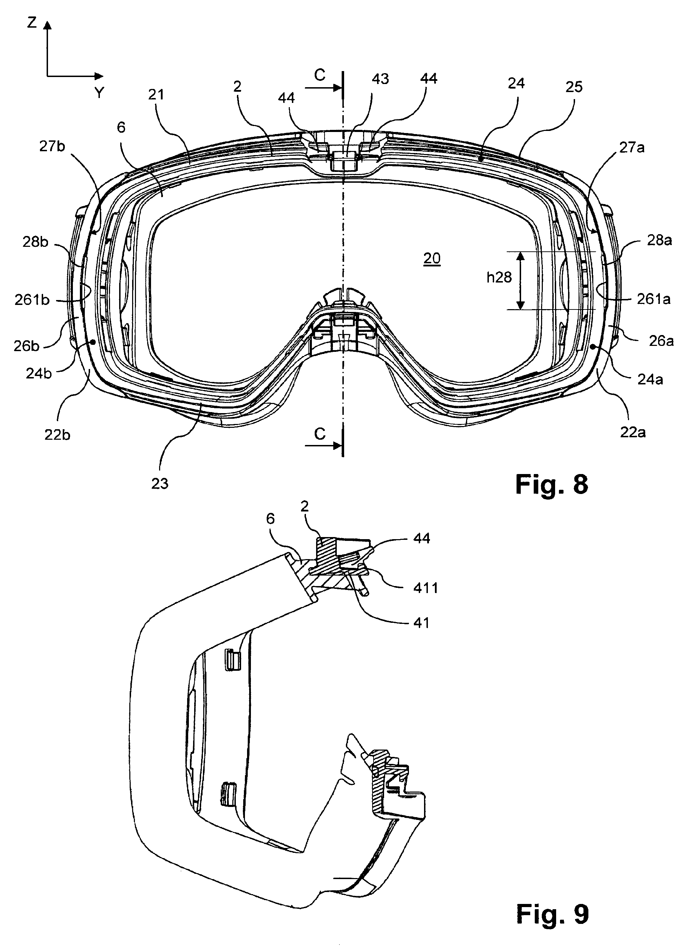

- la figure 8 est une vue de face du masque sans écran ;
- la figure 9 est une vue en coupe selon C-C de la figure 8 ;

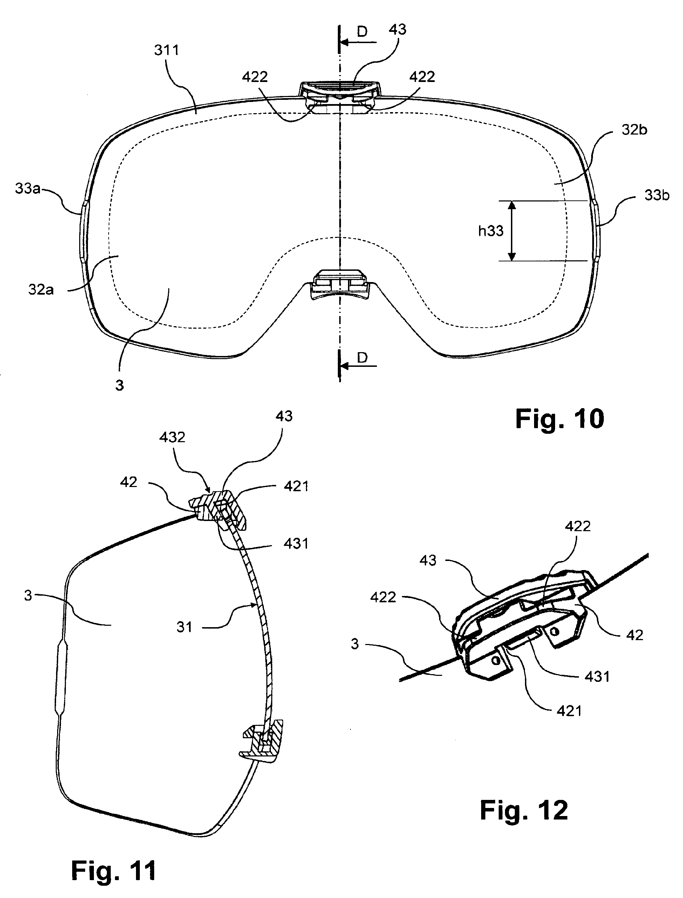

- la figure 10 est une vue arrière de l'écran seul ;
- la figure 11 est une vue en coupe selon D-D de la figure 10 ;
- la figure 12 est une vue en perspective arrière d'une partie de l'écran seul ;
- la figure 13 est une vue en perspective arrière d'un écran d'un masque selon un deuxième mode de réalisation de l'invention ;
- la figure 14 est une vue en perspective avant du châssis du deuxième mode de réalisation du masque ;
- la figure 15 est une vue éclatée selon une coupe horizontale médiane du masque selon le deuxième mode de réalisation.

Le masque 1 comprend un châssis 2 supportant un écran de protection oculaire 3. Une sangle, non représentée, est fixée aux extrémités latérales du châssis 2. La sangle et le châssis 2 forment une boucle destinée à entourer le crâne de l'utilisateur pour assurer le maintien en place du masque.

Dans la suite de la description, il sera fait usage de termes tels que « horizontal », « vertical », « transversal », « supérieur », « inférieur », « latéral », « haut », « bas », « droite », « gauche », « avant », « arrière », « devant », « derrière ». Ces termes doivent être interprétés en fait de façon relative en relation avec la position normale que le masque occupe sur le visage de l'utilisateur quand il se tient debout, tête droite. Par « arrière », on désigne les parties orientées côté tête / yeux de l'utilisateur. On utilisera l'indice « a », en référence aux éléments de la partie gauche du masque et l'indice « b », en référence aux éléments de la partie droite du masque.

On utilisera également un repère dont la direction avant/arrière correspond à l'axe X, la direction transversale ou droite/gauche correspond à l'axe Y et la direction verticale ou haut/bas correspond à l'axe Z.

Dans cet exemple, le masque comprend également une jupe 6 fixée sur une partie arrière du châssis 2. De manière classique, un élément de confort, non représenté, est fixé sur la partie arrière de la jupe. La jupe 6 est constituée d'un matériau souple, déformable, permettant de s'adapter à la morphologie du visage. Il peut être réalisé, par exemple, en polyuréthanne (PU). L'élément de confort est destiné à venir en contact avec le visage. Il est constitué d'un matériau agréable en contact. Il peut être réalisé, par exemple, en mousse en polyuréthanne (PU).

Selon ce mode de réalisation, le châssis 2 forme un cadre délimitant une ouverture 20 destinée à être obturée par l'écran oculaire 3. Cette ouverture 20 couvre, quand le masque est porté, une zone de vision s'étendant d'une tempe à l'autre, du front aux pommettes. Le châssis 2 se présente sous une forme globalement rectangulaire délimitée par un montant supérieur 21, un montant latéral gauche 22a, un montant latéral droit 22b et un montant inférieur 23. Le montant inférieur 23 reproduit un « V » inversé dans sa zone centrale afin d'épouser la forme du nez.

Dans cet exemple, le châssis est réalisé dans un matériau rigide ce qui permet d'avoir un bon support et un bon maintien de l'écran. De plus, cela facilite la déformation de l'écran lors de sa mise en place comme nous le verrons par la suite. Préférentiellement, le matériau présente un module d'élasticité supérieur à 1 000 MPa. Par exemple, ce peut être un PolyAmide (PA), un PolyCabonate (PC), un Acrylonitrile Butadiène Styrène (ABS), chargé fibres ou non.

Le châssis 2 comprend une surface d'appui 24, orientée vers l'avant, prévue pour recevoir une zone périphérique 311 d'une face arrière 31 de l'écran 3. La surface d'appui 24 est sensiblement verticale, voire légèrement bombée, de manière à reproduire le profil de la zone périphérique 311 de l'écran. La surface d'appui 24 se compose de plusieurs surfaces d'appuis ménagées sur les montants supérieurs, latéraux et inférieurs du châssis. Ainsi, les montants latéraux 22a, 22b intègrent les parties latérales 24a, 24b de la surface d'appui 24. La surface d'appui 24 est, par ailleurs, délimitée par un rebord extérieur 25. Ce rebord 25 est dimensionné et positionné de sorte à permettre le calage vertical et transversal de l'écran lorsque celui-ci est assemblé sur le châssis. En conséquence, pour une section sagittale donnée, selon un plan XZ, la distance verticale entre le rebord supérieur et le rebord inférieur est légèrement supérieure à la hauteur de l'écran, lorsqu'il est assemblé au châssis. De même, pour une section horizontale donnée, selon un plan XY, la distance transversale entre les rebords latéraux est légèrement supérieure à la largeur de l'écran, lorsqu'il est assemblé au châssis.

De chaque côté du châssis, le montant latéral 22a, 22b comprend une paroi 26a, 26b disposée en vis-à-vis de la partie 24a, 24b de la surface d'appui, à une distance d26. Cette distance d26 est légèrement supérieure à l'épaisseur e de l'écran 3, au niveau des bords latéraux du pourtour de l'écran. Chaque paroi 26a, 26b est fixée sur la face externe, orientée vers l'avant, d'une partie latérale du rebord extérieur 25.

Ainsi, chaque partie latérale du châssis supporte un logement d'accroche 27a, 27b défini par une paroi 26a, 26b, une partie latérale 24a, 24b de la surface d'appui 24 et une partie du rebord extérieur 25. Chaque logement d'accroche 27a, 27b est prévu pour recevoir un bord latéral 32a, 32b de l'écran 3 lorsque celui-ci est assemblé, par déformation, au châssis.

Dans ce mode de réalisation, chaque logement d'accroche 27a, 27b comprend, en plus, un dégagement 28a, 28b, au niveau du rebord extérieur 25. Le dégagement 28a, 28b correspond à une ouverture, d'une hauteur h28, réalisée dans la zone centrale d'une partie latérale associée du rebord extérieur 25.

L'écran 3 reprend sensiblement la forme de l'ouverture 20 mais avec une légère homothétie, l'agrandissant, pour que l'écran puisse prendre appui sur la surface d'appui 24 du châssis 2.

Dans cet exemple, l'écran présente deux zones de vision 34 destinées à venir en vis-à-vis des yeux de l'utilisateur. Les deux zones de vision 34 sont raccordées ensemble par une partie médiane 35. La partie médiane 35 est destinée à venir à l'aplomb du nez de l'utilisateur. Ainsi, l'écran 3 forme une paroi continue s'étendant entre les zones de vision 34 en passant par la partie médiane 35, afin d'optimiser l'angle de vision de l'utilisateur au niveau de la partie médiane. L'écran 3 couvre donc les deux yeux de l'utilisateur lorsqu'il est assemblé sur le châssis.

Dans cet exemple, chaque bord latéral 32a, 32b de l'écran 3 comprend une extension transversale 33a, 33b s'étendant sur une hauteur h33. Cette extension transversale 33a, 33b est destinée à s'insérer dans le dégagement 28a, 28b du logement d'accroche 27a, 27b correspondant. Ainsi, la hauteur h33 de l'extension transversale 33a, 33b est légèrement inférieure à la hauteur h28 du dégagement 28a, 28b.

En variante, la construction peut être inverse. L'écran comprend une découpe concave. Le logement d'accroche comprend une forme convexe complémentaire.

L'écran 3 comprend un ou plusieurs panneaux optiques permettant de protéger les yeux de l'utilisateur notamment contre le rayonnement solaire. La forme de l'écran est généralement sensiblement sphérique ou sensiblement cylindrique. La face arrière 31 de l'écran, orientée en direction de l'utilisateur, est concave et présente un rayon de courbure Re par rapport au centre de la sphère ou l'axe vertical du cylindre. La face arrière 31 comprend une zone périphérique 311 destinée à venir en contact avec la surface d'appui 24 du châssis.

La surface d'appui 24 du châssis 2 est également sensiblement sphérique ou sensiblement cylindrique. Ainsi, cette surface d'appui 24, orientée en direction de l'écran, est convexe et présente un rayon de courbure Rc par rapport au centre de la sphère ou l'axe vertical du cylindre.

Selon l'invention, la zone périphérique 311 et la surface d'appui 24 ne sont pas complémentaires à l'état repos, c'est-à-dire, lorsque l'écran n'est pas déformé. Cependant, le châssis et l'écran sont dimensionnés de sorte que, lorsqu'on applique un effort sur l'écran, la déformation de l'écran va permettre la conformation de la zone périphérique 311 afin que celle-ci vienne épouser sensiblement la forme de la surface d'appui 24. Pour cela, par exemple, le rayon de courbure Rc du châssis est supérieur au rayon de courbure Re de l'écran ce qui permet d'obtenir une déformation de l'écran assurant la conformation d'une partie 311 de sa face arrière sur la surface d'appui 24.

Dans le mode de réalisation illustré, la zone périphérique 311 et la surface d'appui 24 présentent une différence de rayon de courbure autour d'un axe vertical. En conséquence, cette variation concerne principalement les montants supérieur 21 et inférieur 23 du châssis, et non les montants latéraux 22a, 22b.

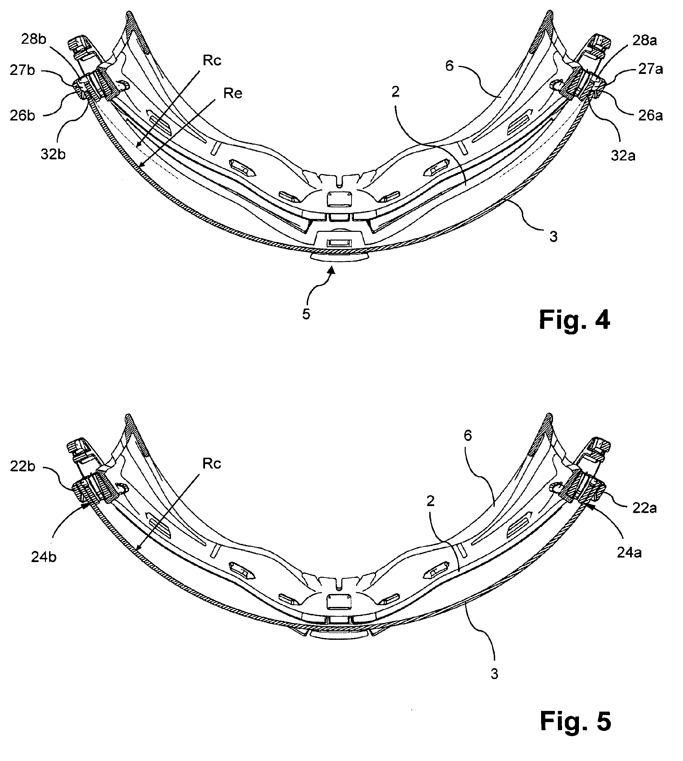

La mise en place de l'écran comprend les étapes suivantes :
Dans un premier temps, comme illustré à la figure 4, l'utilisateur positionne l'écran sur le châssis de sorte que les bords latéraux 32a, 32b de l'écran viennent au contact avec la surface d'appui 24, au niveau des parties latérales 24a, 24b. Dans cette configuration, la zone périphérique 311 n'est pas en contact avec les parties supérieure et inférieure de la surface d'appui, au niveau des montants supérieur et inférieur du châssis. Pour faciliter cette étape de positionnement de l'écran, la largeur L3 de l'écran non déformé, correspondant à la distance transversale L3 (selon l'axe Y) séparant les deux bords latéraux, est toujours inférieure à la distance transversale L26 (selon l'axe Y) séparant les parois externes 26a, 26b délimitant respectivement les logements d'accroche 27a, 27b. Grâce à cet écart dimensionnel, l'écran peut facilement venir en contact avec la surface d'appui, sans interférer avec une partie des logements d'accroche. A noter que pour permettre le passage des extensions transversales 33a, 33b de l'écran, chaque paroi 26a, 26b intègre une découpe 261 a, 261b positionnée en vis-à-vis de l'extension correspondante.
Dans un deuxième temps, comme illustré à la figure 5, l'utilisateur exerce une pression dans la zone centrale de l'écran en direction du châssis. Cette action a pour effet de déformer l'écran de sorte que la zone périphérique 311 se conforme à la surface d'appui. Les bords latéraux 32a, 32b de l'écran s'écartent, l'un de l'autre, selon une direction transversale correspondant à l'axe Y. Chaque bord latéral se déplace latéralement (composante Y) d'une distance supérieure à deux millimètres. Chaque bord latéral 32a, 32b est maintenu en contact contre la surface d'appui et glisse latéralement sur la surface d'appui jusqu'à s'insérer dans un logement d'accroche 27a, 27b correspondant. Cette coopération permet de caler l'écran par rapport au châssis et notamment de le maintenir horizontal, principalement selon l'axe X. De plus, chaque extension transversale 33a, 33b s'insère dans le dégagement 28a, 28b du logement d'accroche 27a, 27b correspondant. Cette coopération permet de caler l'écran par rapport au châssis et notamment de le maintenir verticalement (selon l'axe Z). Par ailleurs, les parties centrales supérieure et inférieure de la zone périphérique 311 se décalent vers l'arrière (composante X) d'une distance supérieure à quatre millimètres. Dans cette configuration, les parties supérieure et inférieure de la zone périphérique 311 viennent en contact ou avec un jeu réduit avec les parties supérieure et inférieure de la surface d'appui, au niveau des montants supérieur et inférieur du châssis. Des moyens de maintien 4, 5, décrits ultérieurement, permettent de maintenir cette configuration d'assemblage.

L'utilisation d'un châssis réalisé avec une matière rigide permet d'améliorer la mise en place de l'écran. En effet, comme il faut exercer une pression sur l'écran pour générer sa déformation, il est préférable que l'écran soit en appui sur une surface rigide. Les bords latéraux 32a, 32b de l'écran peuvent alors mieux glisser sur la surface d'appui 24.

Les moyens de maintien 4, 5 vont maintenant être décrits. Ces deux moyens de maintien présentant un fonctionnement identique, seul le moyen de maintien supérieur 4 va être décrit. Le moyen de maintien inférieur 5 est composé d'éléments analogues mais disposés symétriquement par rapport à un plan horizontal XY.

Dans cet exemple, le moyen de maintien supérieur 4 comprend un clip 41, solidaire du châssis, destiné à coopérer avec une boucle 42, solidaire de l'écran.

Le clip 41 se présente sous la forme d'une patte élastique faisant saillie de la zone centrale du montant supérieur 21, selon l'axe X, en direction de l'écran. La patte élastique comprend, à son extrémité libre, un crochet 411, orienté vers le haut, selon l'axe Y. Ici, c'est une partie du châssis 2 qui forme le clip 41. Le clip 41 et le châssis 2 constituent ainsi une pièce unitaire, une pièce monobloc. Alternativement, le clip est disposé sur une pièce rapportée sur le châssis.

La boucle 42 se présente sous la forme d'un profil en « U » fixé sur la face arrière 31 de l'écran de sorte à créer un orifice 421 orienté selon l'axe vertical Y.

Lorsque l'écran est assemblé sur le châssis, la partie centrale de l'écran se rapproche de la partie centrale du châssis. Le clip 41 se déforme jusqu'à ce que le crochet 411 se positionne en vis-à-vis de l'orifice 421 de la boucle 42. Le clip reprend alors sa configuration au repos. Le crochet 411 pénètre à l'intérieur de l'orifice 421. Le clip coopère alors avec la boucle de sorte que l'écran reste plaqué contre le châssis. En conséquence, l'écran est maintenu déformé contre le châssis par le moyen de maintien. Autrement dit, le moyen de maintien 4 permet de solidariser, selon la direction Y, l'écran avec le châssis.

Pour retirer l'écran, l'utilisateur doit agir sur le clip 41 afin que le crochet 411 se désengage de l'orifice 421. Dans cet exemple, l'écran est muni d'un bouton 43 comprenant un pêne 431 positionné de sorte qu'il puisse coulisser verticalement à l'intérieur de l'orifice 421. Le bouton comprend une surface d'actionnement 432 reliée au pêne et placée au-dessus de celui-ci. La surface d'actionnement est suffisamment large pour recevoir un doigt afin de rendre l'utilisation du bouton ergonomique. Le bouton 43 comprend par ailleurs deux butées permettant de limiter sa course et donc le déplacement vertical du pêne 431. La course du pêne est dimensionnée de sorte que, lorsque le pêne est positionné en bas de sa course, le pêne agit sur le crochet 411 afin qu'il se désengage de l'orifice 421. Par ailleurs, lorsque le pêne est positionné en haut de sa course, le pêne ne perturbe pas le déplacement du crochet à l'intérieur de l'orifice, le crochet devant s'engager suffisamment dans l'orifice pour retenir l'écran. Lorsque le clip 41 vient coopérer avec la boucle 42, cela a pour effet de faire lever le bouton 43. Lorsque l'utilisateur appuie, vers le bas, sur la surface d'actionnement 432, cela provoque le coulissement du pêne jusqu'à ce que le clip ne coopère plus avec la boucle. L'écran peut alors être retiré.

De manière classique, le crochet 411 et le pêne 431 présentent des pentes complémentaires à leur zone de contact interface. Ces pentes permettent d'aider les éléments à se déformer (pour le clip) et/ou se déplacer (pour le pêne).

Du fait que l'écran est maintenu déformé, cela améliore sensiblement la libération de l'écran. En effet, dès que le moyen de maintien 4 est désactivé, c'est-à-dire, que le clip 41 ne coopère plus avec la boucle 42, l'écran va reprendre sa forme initiale, non contrainte. En conséquence, l'écran va naturellement s'écarter du châssis. L'écran peut alors facilement être retiré car il n'y a plus d'éléments susceptibles de le retenir.

Dans des constructions classiques de masque, il arrive que l'utilisateur désactive le moyen de maintien en déformant le châssis, par exemple, en le faisant vriller. Ainsi, si on déforme le châssis de manière à rapprocher les montants inférieur et supérieur, on risque de déplacer verticalement, vers le bas, le clip 41. Or, l'écran étant relativement rigide, la boucle 42 ne se déplace quasiment pas. Il en ressort un risque important que le clip se désengage de la boucle, ce qui entraîne la libération non souhaitée de l'écran.

Pour limiter ce risque, une première solution consiste à utiliser un matériau relativement rigide pour le châssis 2 afin de réduire sa déformation et notamment le déplacement du clip.

Une deuxième solution est implantée dans le mode de réalisation décrit. Le moyen de maintien 4 comprend une butée verticale permettant de solidariser, en translation verticale, selon l'axe Y, le clip avec la boucle, lorsque l'écran est assemblé sur le châssis. Dans cet exemple, la boucle 42 comprend deux plats horizontaux 422, s'étendant vers l'arrière et disposés de part et d'autre de l'orifice 421. Ces deux plats horizontaux 422 sont destinés à s'insérer dans deux rainures horizontales complémentaires 44, ouverts vers l'avant et disposés de part et d'autre du clip 41. Ainsi, lorsque le clip 41 est assemblé avec la boucle 42, les deux plats 422 se positionnent à l'intérieur des rainures 44. Cette coopération forme la butée verticale du moyen de maintien. En effet, si on déforme le châssis vers le bas, les faces supérieures des rainures viennent en contact avec les faces supérieures des plats ce qui entraîne le déplacement vers le bas de l'écran. On n'a pas d'écartement relatif entre le clip 41 et la boucle 42. L'accroche est sécurisée. A l'inverse, si on déforme le châssis vers le haut, les faces inférieures des rainures viennent en contact avec les faces inférieures des plats ce qui entraîne le déplacement vers le haut de l'écran. Là encore, on n'a pas d'écartement relatif entre le clip 41 et la boucle 42. L'accroche est également sécurisée.

On peut envisager d'autres formes de butée verticale pour le moyen de maintien 4.

Selon le mode de réalisation illustré, le masque est muni d'un deuxième moyen de maintien 5, positionné dans la zone centrale du montant inférieur 23 du châssis 2, au sommet du « V » inversé prévu pour épouser la forme du nez. Ce deuxième moyen de maintien 5 présente les mêmes caractéristiques que le premier moyen de maintien 4, si ce n'est que la construction est inversée par rapport à un plan horizontal XY. De même, le dimensionnement du second moyen de maintien est adapté compte tenu des contraintes géométriques liées à son emplacement, notamment en ce qui concerne le châssis.

L'utilisation de deux moyens de maintien 4, 5 apporte plus de stabilité pour maintenir l'écran déformé, contre le châssis. De plus, cela permet une manipulation plus ergonomique de l'écran. Il est, en effet, naturel de prendre l'écran entre le pouce et l'index au niveau des zones centrales des bords supérieur et inférieur de l'écran. Par ailleurs, cela permet d'éviter que l'utilisateur touche directement les panneaux optiques et donc ne salisse ou abime ceux-ci.

Alternativement, le masque peut ne comprendre qu'un seul moyen de maintien. Par exemple, il peut placer l'écran dans une rainure inférieure et faire pivoter l'écran pour bloquer le haut de l'écran. Le masque peut également comprendre plus de deux moyens de maintien. De même, les moyens de maintien ne sont pas nécessairement disposés dans une zone centrale supérieure ou inférieure du châssis.

Dans cet exemple, le clip est porté par le châssis et la boucle par l'écran. Cette construction peut être inversée, le clip étant alors porté par l'écran et la boucle par le châssis. De même, le crochet peut également être orienté vers le bas au lieu d'être orienté vers le haut, comme illustré dans les figures.

Pour réaliser l'invention, d'autres moyens de maintien peuvent être appliqués. Cette solidarisation peut être faite par des aimants, par des boutons pression, par un verrou mobile, par des bandes adhésives type VELCRO®...

Dans le mode de réalisation illustré, le masque comporte deux logements d'accroche, supportés respectivement par un montant latéral du châssis. En variante, le masque peut ne comprendre qu'un seul logement d'accroche. L'écran peut pivoter autour d'un axe vertical latéral et se déformer latéralement uniquement d'un côté pour venir se loger dans le logement d'accroche. Eventuellement, il peut y avoir davantage de logements d'accroches.

Par ailleurs, les moyens d'accroches ne sont pas nécessairement positionnés sur les montants latéraux du châssis. Dans une variante, les moyens d'accroche sont placés sur le montant supérieur et/ou le montant inférieur du châssis.

On peut envisager d'autres formes de réalisation des logements d'accroche. Par exemple, l'écran peut supporter un crochet destiné à coopérer avec une boucle solidaire du châssis.

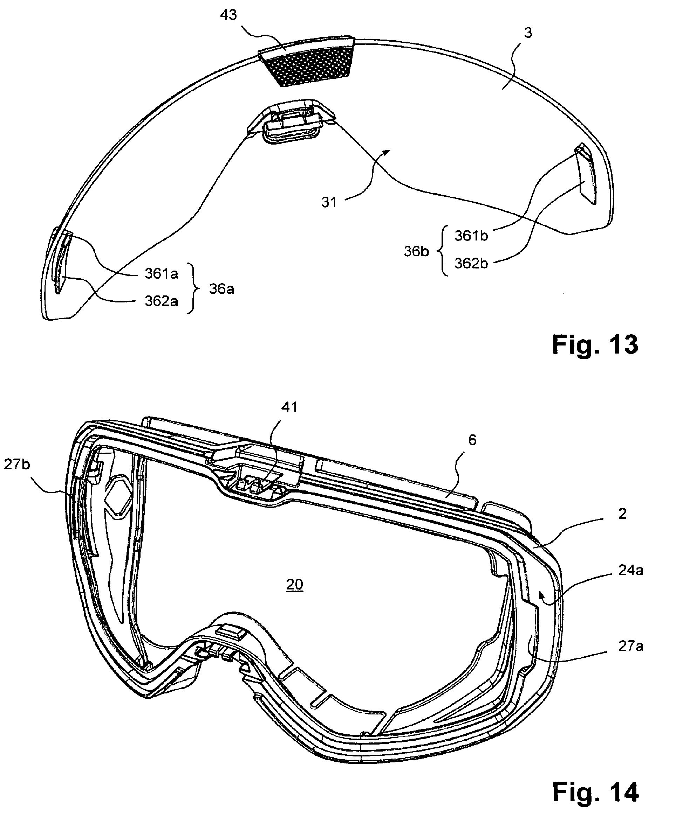

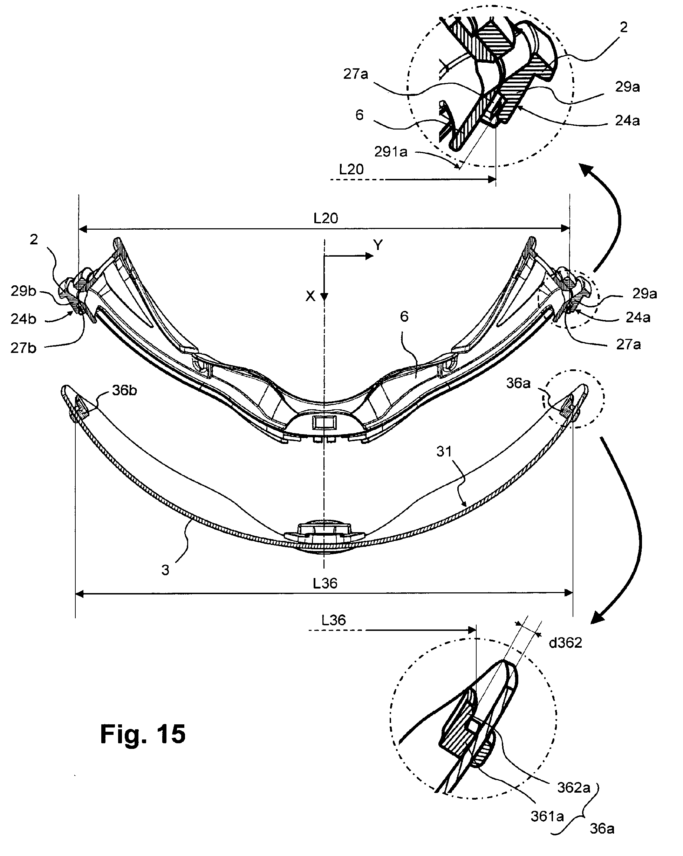

Les figures 13 à 15 illustrent un deuxième mode de réalisation d'un masque selon l'invention. Dans ce mode de réalisation, le moyen d'accroche de l'écran sur le châssis de type crochet/boucle.

Dans cet exemple, un insert 36a, 36b est fixé sur chaque bord latéral 32a, 32b de l'écran 3. Cet insert 36a, 36b s'étend depuis la face arrière 31 de l'écran. Chaque insert 36a, 36b comprend une extension 361 a, 361 b faisant saillie de la face arrière 31, selon une direction sensiblement normale à la face arrière, et un retour 362a, 362b s'étendant depuis l'extrémité libre de l'extension 361 a, 361 b en direction d'une extrémité latérale de l'écran correspondante, en s'éloignant de la partie médiane 35 de l'écran. Chaque retour 362a, 362b définit ainsi une plaque sensiblement parallèle à la face arrière 31 de l'écran. Cette plaque est positionnée à une distance d362 de la face arrière 31. Ainsi, chaque insert 36a, 36b forme un crochet en forme de « L ».

Les inserts 36a, 36b sont fixés sur l'écran par tout moyen approprié. Ce peut être par des vis, du collage, de la soudure ultrason. Alternativement, l'insert 36a, 36b n'est pas une pièce rapportée sur l'écran mais est réalisée par l'écran lui-même. Dans ce cas, le crochet est réalisé par une mise en forme particulière de l'écran, par exemple, par déformation locale ou par moulage.

Comme le mode de réalisation précédent, l'écran 3 et le châssis 2 sont munis de moyens de maintien analogues permettant de maintenir la déformation de l'écran lorsque celui-ci est assemblé sur le châssis.

Pour pouvoir fixer l'écran au châssis conformément à l'invention, le châssis comprend donc un logement d'accroche 27a, 27b adapté, sur chaque montant latéral 22a, 22b. Dans cet exemple, chaque logement d'accroche 27a, 27b est délimité par la face arrière 291 a, 291 b d'une paroi latérale 29a, 29b du châssis 2 portant une surface d'appui 24a, 24b de l'écran et délimitant latéralement l'ouverture 20 du châssis. Le logement d'accroche peut donc être défini uniquement par cette paroi latérale. Alternativement, on peut prévoir un décrochement dans la face arrière 291 a, 291 b de cette paroi latérale 29a, 29b afin de recevoir une partie 362a, 362b de l'insert correspondant 36a, 36b. Le logement d'accroche est alors défini par la face arrière 291 a, 291 b de la paroi latérale 29a, 29b et le décrochement aménagé. Ce décrochement peut typiquement permettre de limiter le déplacement de l'écran selon d'autres directions comme, par exemple, la direction verticale.

L'assemblage de l'écran sur le châssis comprend les étapes suivantes. Dans un premier temps, on approche l'écran 3 du châssis selon une direction X, sensiblement horizontale. Les inserts 36a, 36b passent alors à travers l'ouverture 20 du châssis 2. Puis, la surface périphérique 311 de la face arrière 31 de l'écran 3 vient en contact avec surface d'appui 24 du châssis. En poursuivant le rapprochement, l'écran se déforme du fait du contact avec le châssis. Les bords latéraux 32a, 32b glissent transversalement le long de la surface d'appui 24a, 24b ce qui a pour effet d'écarter transversalement les inserts 36a, 36b, l'un de l'autre. Ainsi, chaque retour 362a, 362b vient se placer en vis-à-vis de la paroi latérale 29a, 29b correspondante du châssis, du côté de la face arrière 291 a, 291 b. On enclenche alors les moyens de maintien 4, 5 permettant de maintenir cet état déformé de l'écran. L'écran est alors verrouillé selon la direction d'assemblage X. Dans un sens, la surface périphérique 311 de l'écran bute contre la surface d'appui 24 du châssis. Dans l'autre sens, chaque insert 36a, 36b bute contre la face arrière 291 a, 291 b d'une paroi latérale 29a, 29b du châssis. Le déplacement de l'écran est ainsi bloqué dans les deux sens, selon la direction d'assemblage X.

Avantageusement, la largeur L20 de l'ouverture 20 du châssis est légèrement plus grande que la distance L362 mesurée entre les deux extrémités libres des retours 362a, 362b des inserts 36a, 36b lorsque l'écran n'est pas déformé. Cela permet de faciliter la mise en place de l'écran sur le châssis du fait que les inserts peuvent librement s'introduire dans l'ouverture 20 du châssis. Selon une variante, on peut accepter que la largeur L20 soit légèrement plus petite que la distance L362. En effet, avec une légère interférence, on peut tout de même placer l'écran sans nécessiter un effort d'assemblage important de l'utilisateur. A noter que l'écart entre la largeur L20 et la distance L362 ne doit pas être trop important car il faut impérativement que la distance L362, après déformation de l'écran, soit plus grande que la largeur L20 afin d'assurer le verrouillage de l'écran. Le dimensionnement de l'écran et du châssis doit donc prendre en compte ces exigences.

Avec ce mode de réalisation, contrairement au mode de réalisation précédent, il n'y a plus besoin de parois « 26 », disposée en vis-à-vis de la partie de la surface d'appui 24, à une distance « d26 » au niveau des montants latéraux du châssis. La structure du châssis est ainsi plus simple. Le châssis peut alors être réalisé avec un moule plus simple et économique.

Ces logements d'accroche 27a, 27b peuvent, ici, être considérés comme des boucles destinées à coopérer avec les crochets/inserts.

Ainsi, chaque insert 36a, 36b est destiné à coopérer avec le logement d'accroche correspondant 27a, 27b, lorsque l'écran est déformé, de sorte à maintenir solidaire l'écran avec le châssis, selon au moins une direction d'assemblage X.

Dans cet exemple, l'écran 3 est illustré avec une seule paroi. Alternativement, l'écran peut comprendre plusieurs parois, celles-ci pouvant être espacées par une couche d'air. Par exemple, l'écran peut comprendre une paroi destinée à s'insérer à l'intérieur de l'ouverture 20 du cadre. Cette paroi supplémentaire présente alors une surface inférieure à celle de la paroi principale de l'écran. Elle se superpose à la paroi principale du côté interne. Dans ce cas, les inserts sont fixés sur la couche principale externe.

Dans ce cas, les inserts peuvent faire saillie vers l'intérieur depuis une de ces couches.

Selon un mode de réalisation, le châssis forme intégralement le logement d'accroche. Le châssis et le logement d'accroche constituent alors une pièce unitaire, une pièce monobloc. Alternativement, le logement d'accroche comprend des éléments rapportés au châssis, comme par exemple, la paroi 26.

Comme précisé précédemment, le logement d'accroche est apte à coopérer avec l'écran uniquement lorsque l'écran est déformé. Au sens de l'invention, cette coopération avec l'écran permet de maintenir solidaire l'écran avec le châssis, selon au moins une direction d'assemblage X. Il est à noter que, pour certains modes de réalisation, l'écran peut être en contact avec une partie du logement lors de la mise en place de l'écran sans pour autant que ce contact assure le maintien ferme de l'écran sur le châssis. Dans cette configuration, l'écran peut facilement se retirer du châssis. L'invention réside dans le fait que le verrouillage de l'écran pour assurer son maintien est réalisé suite à une déformation de l'écran par l'utilisateur. Ainsi, c'est lorsque l'écran est déformé que celui-ci est maintenu solidaire du châssis de manière ferme. En conséquence, il est nécessaire que le masque dispose de moyens de maintien permettant de maintenir cet état déformé de l'écran.

L'invention n'est pas limitée à ces modes de réalisation. Il est possible de combiner ces modes de réalisation.

L'invention n'est pas limitée aux modes de réalisation précédemment décrits mais s'étend à tous les modes de réalisation couverts par les revendications annexées.

### Nomenclature

- 1-: Masque
- 2-: Châssis
- 20-: Ouverture
- 21-: Montant supérieur
- 22a, 22b-: Montant latéral
- 23-: Montant inférieur
- 24, 24a, 24b-: Surface d'appui
- 25-: Rebord
- 26a, 26b-: Paroi
- 261a, 261b-: Découpe
- 27a, 27b: Logement d'accroche
- 28a, 28b: Dégagement
- 29a, 29b: Paroi latérale
- 291 a, 291b: Face arrière
- 3-: Ecran
- 31-: Face arrière
- 311-: Zone périphérique
- 32a, 32b-: Bord latéral
- 33a, 33b-: Extension transversale
- 34-: Zone de vision
- 35-: Partie médiane
- 36-: Insert
- 361-: Extension
- 362-: Retour
- 4-: Moyen de maintien supérieur
- 41-: Clip
- 411-: Crochet
- 42-: Boucle
- 421-: Orifice
- 422-: Plat horizontal
- 43-: Bouton
- 431-: Pêne
- 432-: Surface d'actionnement
- 44-: Rainure horizontale
- 5-: Moyen de maintien inférieur
- 6-: Jupe

## Revendications

1. Masque de protection oculaire (1) pour la pratique de sports en plein air comprenant :
- un écran déformable (3),
- un châssis rigide (2) comprenant au moins un logement d'accroche (27a, 27b) destiné à coopérer avec l'écran afin de maintenir solidaire l'écran avec le châssis, selon au moins une direction d'assemblage (X),
**caractérisé en ce que**
le logement d'accroche est apte à coopérer avec l'écran uniquement lorsque l'écran est déformé,
et **en ce que**
le masque comprend au moins un moyen de maintien (4, 5) permettant de maintenir la déformation de l'écran lorsque celui-ci est assemblé sur le châssis.

2. Masque de protection oculaire (1) selon la revendication 1, **caractérisé en ce que** le châssis est constitué d'un matériau présentant un module d'élasticité supérieur à 1 000 MPa,

3. Masque de protection oculaire (1) selon l'une des revendications précédentes, le châssis est constitué d'un matériau parmi le PolyAmide (PA), le PolyCabonate (PC), l'Acrylonitrile Butadiène Styrène (ABS).

4. Masque de protection oculaire (1) selon l'une des revendications précédentes, **caractérisé en ce que** le châssis comprend une surface d'appui (24, 24a, 24b) contre lequel vient s'appuyer l'écran et **en ce que** le logement d'accroche est délimité par une partie (24a, 24b) de la surface d'appui et une paroi (26a, 26b), disposée en vis-à-vis de la partie de la surface d'appui, à une distance (d26).

5. Masque de protection oculaire (1) selon la revendication précédente, **caractérisé en ce que** la surface d'appui présente un rayon de courbure (Rc) supérieur au rayon de courbure (Re) de la face arrière (31) de l'écran.

6. Masque de protection oculaire (1) selon l'une des revendications précédentes, **caractérisé en ce que** le logement d'accroche comprend un aménagement (28a, 28b) destiné à coopérer avec une forme (33a, 33b) de l'écran de sorte à maintenir solidaire l'écran avec le châssis selon une direction (Z) différente de la direction d'assemblage (X).

7. Masque de protection oculaire (1) selon l'une des revendications précédentes, **caractérisé en ce que** le au moins un moyen de maintien comprend un clip (41).

8. Masque de protection oculaire (1) selon l'une des revendications précédentes, **caractérisé en ce que** le au moins un moyen de maintien (4) est disposé au centre d'un montant supérieur (21) du châssis.

9. Masque de protection oculaire (1) selon la revendication précédente, **caractérisé en ce qu'**il comprend un deuxième moyen de maintien (5) disposé au centre d'un montant inférieur (23) du châssis.

10. Masque de protection oculaire (1) selon l'une des revendications précédentes, **caractérisé en ce qu'**il comprend deux logements d'accroches (27a, 27b) disposés respectivement sur un montant latéral (22a, 22b) du châssis.

11. Masque de protection oculaire (1) selon la revendication précédente, **caractérisé en ce que** l'écran et le châssis sont dimensionnés de sorte que chaque bord latéral (32a, 32b) de l'écran se déforme transversalement, entre une position libre de l'écran et une position de l'écran assemblé sur le châssis, d'une distance supérieure à deux millimètres.

12. Masque de protection oculaire (1) selon l'une des deux revendications précédentes, **caractérisé en ce que** la largeur (L3) de l'écran non déformé, correspondant à la distance transversale séparant les deux bords latéraux, est toujours inférieure à la distance transversale (L26) séparant les parois externes (26a, 26b) délimitant respectivement les logements d'accroche (27a, 27b).

13. Masque de protection oculaire (1) selon l'une des revendications précédentes, **caractérisé en ce que** l'écran (3) couvre donc les deux yeux de l'utilisateur lorsqu'il est assemblé sur le châssis (2).

14. Masque de protection oculaire (1) selon l'une des revendications précédentes, **caractérisé en ce que** l'écran (3) comprend des inserts (36a, 36b) s'étendant depuis la face arrière (31) de l'écran, chaque insert (36a, 36b) étant destiné à coopérer avec le logement d'accroche correspondant (27a, 27b), lorsque l'écran est déformé, de sorte à maintenir solidaire l'écran avec le châssis, selon au moins une direction d'assemblage (X).

15. Masque de protection oculaire (1) selon l'une des revendications précédentes, **caractérisé en ce que** la face arrière (31) de l'écran comprend une zone périphérique (311) destinée à venir en contact avec une surface d'appui (24) du châssis, la zone périphérique étant prévue pour épouser sensiblement la forme de la surface d'appui lorsque l'écran est déformé.
